# EUROPÄISCHE PATENTSCHRIFT

(11) **EP 0 973 561 B1**
(45) Veröffentlichungstag und Bekanntmachung des Hinweises auf die Patenterteilung: **24.09.2003**
(21) Anmeldenummer: 98919106.9
(22) Anmeldetag: 17.03.1998
(51) Int. Cl.: A61L 24/00

(54) **ANTIBIOTIKUMHALTIGE KNOCHENZEMENTPASTE**
BONE CEMENT PASTE CONTAINING AN ANTIBIOTIC
CIMENT OSSEUX PATEUX CONTENANT UN ANTIBIOTIQUE

(30) Priorität: 01.04.1997 DE 19713229
(43) Veröffentlichungstag der Anmeldung: 26.01.2000
(73) Patentinhaber: MERCK PATENT GmbH, 64293 Darmstadt (DE)
(72) Erfinder: WENZ, Robert, D-61206 Wöllstadt (DE); NIES, Berthold, D-64407 Fränkisch-Crumbach (DE)
(86) Internationale Anmeldenummer: EP9801546
(87) Internationale Veröffentlichungsnummer: WO98043685

(56) Entgegenhaltungen:
- EP-A- 0 147 021
- EP-A- 0 242 672
- US-A- 4 291 013

## Beschreibung

Die Erfindung betrifft eine injizierbare aushärtbare Knochenzementpaste auf Basis von bioresorbierbarem die als kationisches Antibiotikum Gentamycinsulfat, Tobramycinsulfat, Amikacinsulfat oder Netilmycinsulfat enthält, wobei der ausgehärtete bioabbaubare Knochenzement das Antibiotikum in depotartiger Wirkung über einen langen Zeitraum in biologisch wirksamen Konzentrationen von 40 bis 50 mg/g Zementpulver freisetzt. Die erfindungsgemäßen Knochenzemente sind geeignet zur Behandlung von infektiösen Entzündungen des Knochens und des Knochenmarks, insbesondere als Folge von Knochendefekten und Knochenfrakturen.

Natürlich vorkommendes Knochenmineral besteht aus Calciumphosphat von Hydroxylapatit-Struktur. Die Zusammensetzung von Knochenmineralien entspricht dabei allerdings nicht der idealen stöchiometrischen Zusammensetzung von kristallinem Hydroxylapatit (Ca₁₀(PO₄)₆(OH)₂), sondern weist in der Regel eine nicht-stöchiometrische Zusammensetzung auf, die durch den Einbau von anderen Anionen wie Carbonat oder Hydrogenphosphat anstelle von Orthophosphat aber auch von anderen Kationen wie Natrium, Kalium oder Magnesium anstelle von Calcium verursacht wird.

Seit einigen Jahren ist man in der Lage, synthetisches Knochenersatzmaterial auf Basis von Hydroxylapatit-ähnlichen Calciumphophatverbindungen herzustellen, das aufgrund seiner qualitativen und strukturellen Ähnlichkeit dem natürlichen Knochen nahe kommt. Damit können die bekannten Nachteile vermieden werden, die durch die Beschaffung von natürlichem autogenen oder allogenen Knochen entstehen können.
Die Eigenschaften dieser synthetischen Hydroxylapatite, insbesondere die physiologische Akzeptanz, die geforderte Bioresorbierbarkeit und die Fähigkeit durch neu generiertes natürliches Knochengewebe, bzw. Stimulation des Wachstums desselben, ersetzt zu werden, hängen von dem mehr oder weniger stark ausgeprägten Kristallisationsgrad, der Partikelgröße sowie der Porösität ab, die bei der Herstellung erzielt werden können. Ferner haben diese Materialien den Vorteil, daß sie mechanischen Belastungen praktisch genau so gut widerstehen wie der natürliche Knochen, was ihren Einsatz bei größeren Knochendefekten oder -frakturen nahelegt.

Solche Materialien sind beispielsweise bekannt aus der EP 0416 761, der US 4,880,610, der US 5,053,212, der EP 0664 133, der EP 0 543 765 oder der WO 96/36562. Aus der WO 96/36562 ist ein Hydroxylapatit-Knochenzement bekannt, welcher aufgrund seiner nahezu amorphen Struktur eine ausgezeichnete Bioresorbierbarkeit und trotz seiner Porösität eine gute mechanische Stabilität aufweist. Dieses Material, α-BSM genannt, läßt sich in Form von aushärtbaren Pasten herstellen, die leicht mittels einer Spritze in den defekten Knochen eingebracht werden können.

### Stand der Technik:

Die U.S. 4,291,013 beschreibt eine im Körper resorbierbare geformte Masse auf Basis von Kollagen, ihre Herstellung und ihre Verwendung in der Medizin, insbesondere als chirurgisches Material und/oder als Wirkstoffdepot. Die Mischungen, die optional auch Calciumphosphatzement enthalten können, werden zu Tabletten, Pillen, Granulaten, Plättchen oder Kugeln geformt.

Die EP-147021 beschreibt eine pharmazeutische Zusammensetzung zum Einfüllen in Knochenhöhlen, umfassend eine wässrige Paste, enthaltend gepulvertes resorbierbares Calciumphosphat und eine antibakterielle Substanz zusammen mit einem oder mehreren resorbierbaren Bindemitteln. Bevorzugt wird ß-TCP (Tricalciumphosphat) eingesetzt.

Die EP-242672 beschreibt ein resorbierbares, poröses Tricalciumphosphat, das als Zusatzstoff zu Implantationsmaterialien auf der Basis von Polyacrylaten und oder PMMA geeignet ist, und dessen Poren mit einem körperverträglichen, resorbierbaren Füllstoff gefüllt sind, wobei der Füllstoff aus mindestens zwei Bestandteilen zusammengesetzt ist, wobei ein Bestandteil ein Antibiotikum ist.

Bei Verletzungen des Knochens, insbesondere bei offenen Brüchen oder Trümmerfrakturen ist in der Regel ein erhebliches Infektionsrisiko gegeben. Diesem kann prinzipiell durch Zugabe von Antibiotika begegnet werden. Eine lokale, einmalige und in der Dosis limitierte Applikation bei der chirurgischen Versorgung der Knochenfraktur bewirkt lediglich, daß nur ein kleiner Teil der im Knochengewebe angesiedelten und aufgrund unzureichender Durchblutung sehr schlecht zugänglichen Infektionskeime abtötet wird. Als Folge können daher teilweise schwere und lebensbedrohliche Entzündungen des Knochenmarks oder des Knochengewebes auftreten.
Um dies zu vermeiden, werden beispielsweise antibiotikahaltige Polymerkugeln (DE 23 20 373, DE 26 51 441) in die Wund- bzw. Markraumhöhle bei der Erstversorgung des defekten Knochens eingebracht. Aus diesem Polymermaterial kann das Antibiotikum über einen längeren Zeitraum hinausdiffundieren und so in ausreichender Konzentration seine keimtötende Wirkung entfalten. Der Nachteil dieser Methode ist, daß das für die Freisetzung des Antibiotikums geeignete Polymermaterial zumeist nicht oder ungenügend biologisch abbaubar ist, so daß es nach der Heilung des Knochens wieder mittels einer erneuten Operation entfernt werden muß. Das Einbringen des Antibiotikums direkt in den zur Verwendung kommenden synthetischen Knochenzement scheiterte bislang daran, daß keine Möglichkeiten gefunden wurden, das Antibiotikum in biologisch wirksamen Konzentrationen unter Einbeziehung einer toxologisch noch vertretbaren Gesamtdosis über einen ausreichend langen Zeitraum verzögert aus dem Zement freizusetzen. In der Regel findet in solchen Fällen eine sehr rasche Freisetzung der gesamten Antibiotikamenge statt.

Es wurde nun überraschend gefunden, daß Knochenzemente auf Basis von bioresorbierbarem α-BSM die kationischen Antibiotika, Gentamycinsulfat, Tobramycinsulfat, Amikacinsulfat oder Netilmycinsulfat über einen langen Zeitraum in biologisch wirksamen Konzentrationen von 40 bis 50 mg/g Zementpulver kontinuierlich aus dem ausgehärteten Knochenzement in die Markraumhöhle und das umgebende Gewebe in Art und Weise eines Wirkstoffdepots mit steuerbarer Freisetzung abgegeben werden.
Der große Vorteil eines solchen erfindungsgemäßen Systems ist es, daß ein zweiter gezielter chirurgischer Eingriff zur Entfernung von nicht biologisch abbaubaren Ploymermaterialien, die bislang verbreitet als Wirkstoffdepot für derartige Anwendungen eingesetzt werden, überflüssig wird. Entzündliche Prozesse am Knochenmark oder Knochengewebe können so sehr einfach gleichzeitig mit der Rekonstruktion des Knochens behandelt oder aber erst verhindert werden. Dies ist Zeit sparend, kostengünstig und erspart dem Patienten die Unannehmlichkeiten eines weiteren chirurgischen Eingriffs inklusive stationärer Behandlung.

Erfindungsgemäß sind besonders die Zementmaterialien bevorzugt, die aus der EP 0 543 765 oder der WO 96/36562 bekannt sind, insbesondere aber α-BSM aus der WO 96/36562.

Gegenstand der Erfindung ist somit eine plastische, injizierbare, aushärtbare Knochenzementpaste auf Basis von bioresorbierbarem α-BSM, die als kationisches Antibiotikum-Salz Gentamycinsulfat, Tobramycinsulfat, Amikacinsulfat oder Netilmycinsulfat enthalten. Gegenstand der Erfindung ist ebenfalls ein entsprechendes Wirkstoffdepot für den Einsatz zur Behandlung und Prophylaxe von Osteomyelitis und Ostitis, insbesondere als Folge von Knochendefekten und Knochenfrakturen. Insbesondere für größere Frakturen und Trümmerfrakturen mit offenen und daher leicht infizierbaren Wunden ist das erfindungsgemäße System vorzüglich geeignet

Es wurde gefunden, daß aus der Gruppe der kationischen Antibiotika vor allem die Aminoglycoside Gentamycinsulfat, Tobramycinsulfat, Amikacinsulfat oder Netilmycinsulfat die gewünschten vorteilhaften Freisetzungseigenschaften zeigen. Die Aminoglycoside zeigen nicht nur hervorragende Freisetzungseigenschaften im erfindungsgemäßen Sinn sondern in der Regel auch noch ein breites konzentrationsabhängiges Wirkungsspektrum. Als im Sinne der Erfindung bevorzugtes Aminoglycosid ist Gentamycin zu nennen.

Aus der Reihe der geeigneten Peptid-Antibiotika ist vor allem Polymyxin B hervorzuheben. Ferner zeigt auch Capreomycin, ein β-Lactamase Hemmer die gewünschte erfindungsgemäße Wirkung.

Es wurde ferner gefunden, daß neben der Art des Antibiotikums auch seine Darreichnungsform eine wichtige Rolle spielt. So zeigen nur die entsprechenden Salze die gewünschten Depotwirkungen und Freisetzungscharakteristika. Als Salze eigenen sich prinzipiell anorganische wie organische Salze. Bevorzugte anorganische Salze sind die Sulfate, Phosphate, Hydrogenphosphate und Chloride, während bei den organischen Salzen die Citrate, Acetate und Lactate bevorzugt sind. Besonders vorteilhafte Ergebnisse erzielt man aber mit den entsprechenden Sulfaten. Das ganz besonders bevorzugte Antibiotikum ist demnach Gentamycinsulfat.

Gegenstand der Erfindung ist somit eine injizierbare aushärtbare Knochenzementpaste, bzw. ein entsprechendes Wiekstoffdepot, auf Basis von bioresorbierbarem α-BSM, die als kationisches Antibiotikum-Salz Gentamycinsulfat, Tobramycinsulfat, Amikacinsulfat oder Netilmycinsulfat enthalten.

Erfindungsgemäß wird das entsprechende Knochenzementpulver auf Basis von Calciumphosphat enthaltenden Verbindungen mit einer wäßrigen Lösung des Antibiotikums gegebenenfalls unter Zusatz von Hilfsstoffen wie Stabilisierungs-, Konservierungs-, Binde- und / oder Komplexierungsmitteln vermischt. Es entsteht eine je nach Flüssigkeitsmenge mehr oder weniger viskose Paste, die beispielsweise in eine Spritze aufgenommen werden kann. Gegenstand der Erfindung ist somit ein Verfahren zur Herstellung einer injizierbaren aushärtbaren Knochenzementpaste auf Basis von bioresorbierbarem α-BSM, enthaltend Gentamycinsulfat, Tobramycinsulfat, Amikacinsulfat oder Netilmycinsulfat, dadurch gekennzeichnet, daß man Knochenzementpulver auf Basis von α-BSM mit einer wäßrigen Lösung des Antibiotikums gegebenenfalls unter Zusatz von Hilfsstoffen wie Stabilisierungs-, Konservierungs-, Binde- und / oder Komplexierungsmitteln vermischt.

Altemativ können natürlich auch Antibiotikum und Zementpulver direkt miteinander vermischt werden, bevor die entsprechende Menge Flüssigkeit hinzugegeben wird.

Gegenstand der Erfindung ist femer die Verwendung eines entsprechenden Knochenzementspulvers auf Basis von α-BSM zusammen mit Gentamycinsulfat, Tobramycinsulfat, Amikacinsulfat oder Netilmycinsulfat zur Herstellung einer injizierbaren, aushärtbaren, bioresorbierbaren, α-BSM Knochenzementpaste mit der Fähigkeit, das Antibiotikum nach Aushärtung *in vivo* in biologisch wirksamen Konzentrationen von 40 bis 50 mg/g Zementpulver und über einen langen Zeitraum freizusetzen, für die Behandlung und Prophylaxe von Osteomyelitis und Ostitis, insbesondere als Folge von Knochendefekten oder -frakturen.

In der Regel werden zur Herstellung der Paste 0,3 1,5 ml Flüssigkeit (inklusive aller gelösten oder darin suspendierten Verbindungen) pro etwa 1 g Zementpulver (ggf. inklusive des Antibiotikums) gegeben, je nach Abhängigkeit von der gewünschten Konsistenz und der davon abhängigen Aushärtungszeit. Die Konzentration des Antibiotikums, bezogen auf den Knochenzement beträgt 10 -100 mg / g , vorzugsweise 40 - 50 mg, In Abhängigkeit von der erforderlichen Wirksamkeit und der vorgesehenen Zementmenge. Gesamtdosen von 300 mg Antibiotikum sollten aus Toxizitätsgründen nach Möglichkeit nicht deutlich überschritten werden.

Vorzugsweise wird das Antibiotikum in der Flüssigkeit gelöst. In diesem Fall können zusätzlich bekannte Stabilisierungs- und / oder Konservierungsmittel wie, zum Beispiel, Natriumbenzoat, Editinsäure, EDTA in hierfür übliche Konzentrationen (0.1 - 3 %) eingesetzt werden. Für den Fall, daß die erzeugte Paste mittels einer Spritze appliziert werden soll, können der Flüssigkeit auch Bindemittel zugesetzt werden. So hat sich gezeigt, daß Polyethylenglycol (PEG) eine sehr geschmeidige Paste verursacht und eventuellen Entmischungen beim Leeren der Spritze entgegenwirkt. Erfindungsgemäß können auch andere Hilfsstoffe, die Einfluß auf die Freisetzung haben können, wie zum Beispiel Dextransulfat, beigefügt werden.

Als erfindungsgemäße Flüssigkeiten, die mit dem Knochenzementpulver vermischt werden, eignen sich alle Flüssigkeiten auf Wasserbasis, beispielsweise Phosphatpuffer, Kochsalzlösung, Blut oder Serum. In Fällen, in denen das Antibiotikum-Salz ausreichende Löslichkeit besitzt, können auch polare physiologisch akzeptale organische Lösungsmittel, wie das bereits genannte PEG verwendet werden.

Vor der Anwendung der antibiotikahaltigen Paste durch den Arzt können gemäß der Erfindung die einzelnen Komponenten in einzelnen getrennten Packungen (Pre-Packaging) bereit gestellt werden. Die eine Packung enthält dabei die festen Komponenten (Knochenzementpulver, Knochenzementpulver + Antibiotikum). Dabei können verschiedene Packungsgrößen entsprechend der Größe des zu behandelnden Knochendefektes und /oder entsprechend der erforderlichen Antibiotikumkonzentration (im Fall, daß Zementpulver und Antibiotikum in fester Form vermischt werden) hergestellt und angeboten werden. Die zweite Packung besteht aus der Flüssigkeit, in der in der bevorzugten Ausführungsvorm das Antibiotikum und optional die erwähnten Hilfsstoffe gelöst sind. Auch hier können erfindungsgemäß verschiedene Packungsgrößen (unterschiedliche Flüssigkeitsmengen. bzw. unterschiedliche Antibiotikakonzentrationen) bereit gestellt werden, die auf die unterschiedlich erforderlichen Zementmengen der ersten Komponente abgestimmt sind.

Gegenstand der Erfindung ist somit ein Kit-of-Parts, enthaltend im wesentlichen die folgenden getrennt gepackten Komponenten: (i) eine definierte Menge von Knochenzementpulver auf Basis von Calciumphospat enthaltenden Verbindungen und (ii) eine darauf abgestimmte Flüssigkeitsmenge enthaltend Gentamycinsulfat, Tobramycinsulfat, Amikacinsulfat oder Netilmycinsulfat in einer wirksamen Konzentration ggf. in Anwesenheit von Stabilisierungs-, Konservierungs-, Bindeund / oder Komplexierungsmitteln, wobei die Flüssigkeitsmenge so gewählt ist, daß nach Zusammenbringen der beiden Komponeneten (i) und (ii) eine leicht injizierbare aushärtbare Paste entsteht.
Insbesondere ist Gegenstand der Erfindung ein entspechender Kit-of-Parts, enthaltend die genannten Komponenten in folgenden Einheiten: 1g von (i) und 0.7 ml von (ii) mit einem variabel einstellbaren Antibiotikumgehalt von 10 -100 mg / ml.

Femer ist Gegenstand der Erfindung ein Kit-of-Parts, enthaltend im wesentlichen die folgenden getrennt gepackten Komponenten: (i) eine definierte Menge von Knochenzementpulver auf Basis von Calciumphospat enthaltenden Verbindungen, vermengt mit Gentamycinsulfat, Tobramycinsulfat, Amikacinsulfat oder Netilmycinsulfat in einer biologisch wirksamen Konzentration zwischen 40 und 50 mg / g Zementpulver ggf. in Anwesenheit eines Stabilisierungs-, Konservierungs-, Binde- und / oder Komplexierungsmittels und (ii) eine darauf abgestimmte Flüssigkeitsmenge, die so gewählt ist, daß nach Zusammenbringen der beiden Komponenten (i) und (ii) eine leicht injizierbare aushärtbare Paste entsteht.

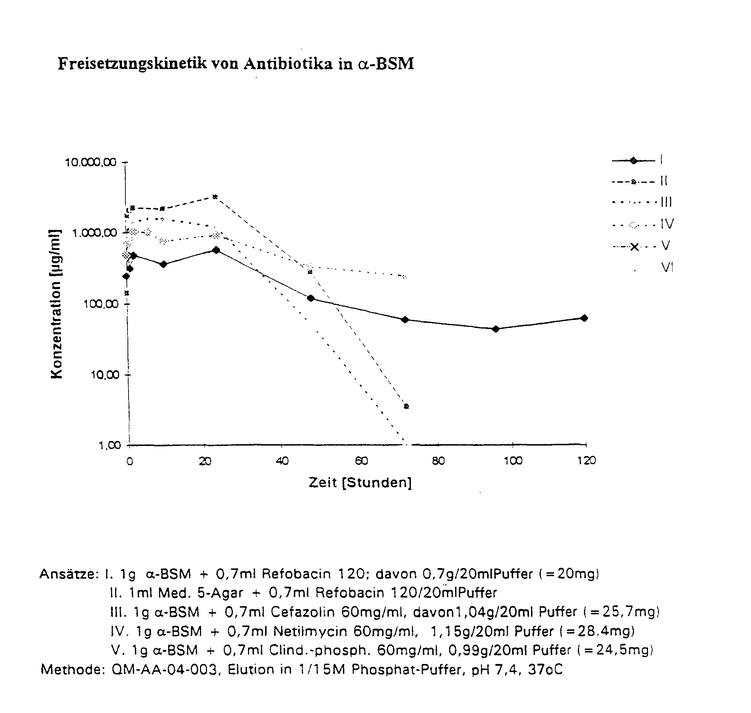

Die erfindungsgemäßen Knochenzementpasten setzen nach Aushärtung kontinuierlich je nach Ausgangsmenge (20 - 60 mg / g Zementpulver) über einen Zeitraum von bis zu 10 Tagen Antibiotikum in einer fast gleichbleibenden Konzentration von etwa 500 - 100 µg / ml frei (Figur 1, Tabelle 1). Dies ist ein Vielfaches der biologisch wirksamen Mindestkonzentration der meisten Antibiotika.
Verwendet man hingegen keine kationischen Antibiotikumsalze, sondern z.B. Cephalosporine, so erfolgt die Freisetzung des Antibiotikums innerhalb weniger Stunden vollständig (Figur 1, Tabelle 1). Da das freigesetzte Antibiotikum in der Regel im Körper rasch weiter verdünnt und abgebaut wird, ist es offensichtlich, daß bei einer solchen Freisetzungskinetik schwer zugängliche und sich erst im Laufe der Zeit entwickelnde Keime nicht erfaßt werden. Wie ferner der Figur und der Tabelle zu entnehmen ist, spielt auch der Träger eine entscheidene Rolle. So wird das erfindungsgemäße Antibiotikum ( z. B. Gentamycinsulfat) ebenso rasch freigesetzt, wenn anstelle der erfindungsgemäßen Hydroxylapatit-ähnlichen Zementpasten anderes Material verwendet wird.
Mit Hilfe der erfindungsgemäßen Knochenzementpasten lassen sich nicht nur Entzündungen des Knochens und des Knochenmarks bei Knochendefekten verhindern, sondern bereits ausgebrochene Entzündungen können zum Abklingen gebracht werden, nachdem der chirurgische Eingriff erfolgt ist.
Figur 1 gibt die Freisetzung verschiedener Antibiotika (Gentamycinsulfat (= Refobacin™), Cefazolin (Cephalosporin) und Netilmycinsulfat aus α-BSM Zement nach Aushärtung in Abhängigkeit von der Zeit wieder. Als Kontrolle dient eine entsprechende Kinetik mit Agar. Tabelle 1 gibt die zugrunde liegenden Einzelwerte wieder

### Beispiel 1:

1 g Zementpulver (α-BSM, WO 96/36562) und 0,7 ml Wasser, in welches 42 mg Gentamycinsulfat gelöst wurden, werden miteinander gemischt. Man erhält dadurch eine Paste, die zu einer Kugel von ca. 1 cm Durchmesser geformt wird. Diese Kugel wird in 20 ml Phosphatpuffer nach Sörensen (pH 7.4) bei 37° C gegeben. Nach etwa 10 Minuten härtet die Zementkugel aus. Zu definierten Zeiten werden Proben aus der Pufferlösung entnommen und die Konzentration des Antibiotikums nach Standardmethoden mikrobiologisch oder chromatographisch bestimmt. Die bestimmten Antibiotikakonzentrationen in µg / ml entsprechen den im Puffer freigesetzten Konzentratuinen.

### Beispiel 2:

Analog Beispiel 1 wird eine zuvor ausgehärtete Kugel aus Agar / Gentamycinsulfat eluiert und die entsprechenden Konzentrationen gemessen.

### Beispiel 3:

Analog Beispiel 1 wird eine α-BSM / Cefazolin Kugel eluiert und die entsprechenden Konzentrationen gemessen.

### Beispiel 4:

Analog Beispiel 1 wird eine α-BSM / Netilmycinsulfat Kugel eluiert und die entsprechenden Konzentrationen gemessen.

**Tabelle 1:**

| **Antibiotica-Elution aus α-BSM** | | | | | | |
|---|---|---|---|---|---|---|
| *µg*/*ml* | | | | | | |
| *Stunden* | *I* | *II* | *III* | *IV* | *V* | *VI* |
| 0 | 245,96 | 139,64 | 478,57 | 484,78 | 0,00 | 0,00 |
| 0,5 | 474,46 | 1.713,10 | 862,82 | 708,39 | 0,00 | 0,00 |
| 1 | 313,41 | 2.036,00 | 1.344,80 | 832_{,}15 | 0,00 | 0,00 |
| 2 | 484,46 | 2.199,20 | 1.387,50 | 1.021,00 | 0,00 | 0,00 |
| 6 | #NV | #NV | 1.563,00 | 1.009,40 | 0,00 | 0,00 |
| 10 | 364,26 | 2.145,80 | 1.554,80 | 751,94 | 0,00 | 0,00 |
| 24 | 565,89 | 3.097,30 | 1.189,10 | 906,52 | 0,00 | 0,00 |
| 48 | 116,19 | 273,63 | 50,16 | 332,69 | 0,00 | 0,00 |
| 72 | 58,00 | 3,46 | 1,00 | 243,59 | 0,00 | 0,00 |
| 96 | 42,77 | | | | 0,00 | 0,00 |
| 120 | 61,41 | | | | 0,00 | |

## Patentansprüche

1. Injizierbare aushärtbare Knochenzementpaste auf Basis von bioresorbierbarem α-BSM, enthaltend ein Arzneimittel bestimmter Konzentration, **dadurch gekennzeichnet, dass** das Arzneimittel Gentamycinsulfat, Tobramycinsulfat, Amikacinsulfat oder Netilmycinsulfat in einer Konzentration von 40 bis 50 mg/ g Zementpulver ist.

2. Wirkstoffdepot, enthaltend eine gemäß Anspruch 1 definierte Knochenzementpaste zur kontinuierlichen Freisetzung des Antibiotikums Gentamycinsulfat, Tobramycinsulfat, Amikacinsulfat oder Netilmycinsulfat in einer Konzentration von 40 bis 50 mg/g Zementpulver und über einen langen Zeitraum für die Behandlung und Prophylaxe von Osteomylitis und Ostitis, insbesondere als Folge von Knochendefekten oder-frakturen.

3. Verwendung eines Knochenzementpulvers auf Basis von bioresorbierbarem α-BSM zusammen mit Gentamycinsulfat, Tobramycinsulfat, Amikacinsulfat oder Netilmycinsulfat zur Herstellung einer injizierbaren, aushärtbaren, bioresorbierbaren, Hydroxylapaptitähnlichen Knochenzementpaste mit der Fähigkeit, das Antibiotikum nach Aushärtung in vivo in einer biologisch wirksamen Konzentration von 40 bis 50 mg/ g Zementpulver und über einen langen Zeitraum freizusetzen, für die Behandlung und Prophylaxe von Osteomyelitis und Ostitis, insbesondere als Folge von Knochendefekten oder -frakturen.

4. Verfahren zur Herstellung einer injizierbaren aushärtbaren Knochenzementpaste auf Basis von bioresorbierbarem α-BSM, enthaltend Gentamycinsulfat, Tobramycinsulfat, Amikacinsulfat oder Netilmycinsulfat, **dadurch gekennzeichnet, dass** man Knochenzementpulver auf Basis von α-BSM mit einer wässrigen Lösung des Antibiotikums gegebenenfalls unter Zusatz von Stabilisierungs-, Konservierungs-, Binde- und /oder Komplexierungsmitteln vermischt.

5. Kit-of-Parts, enthaltend im wesentlichen die folgenden getrennt gepackten Komponenten: (i) eine definierte Menge von Knochenzementpulver auf Basis von α-BSM und (ii) eine darauf abgestimmte Flüssigkeitsmenge enthaltend Gentamycinsulfat, Tobramycinsulfat, Amikacinsulfat oder Netilmycinsulfat in einer biologisch wirksamen Konzentration von 40 bis 50 mg/ g Zementpulver ggf. in Anwesenheit von Stabilisierungs-, Konservierungs-, Bindeund/oder Komplexierungsmitteln, wobei die Flüssigkeitsmenge so gewählt ist, dass nach Zusammenbringen der beiden Komponenten (i) und (ii) eine leicht injizierbare aushärtbare Paste entsteht.

6. Kit-of-Parts nach Anspruch 5, enthaltend die genannten Komponenten in folgenden Einheiten: 1 g von (i) und 0.7 ml von (ii) mit einem variabel einstellbaren Antibiotikumgehalt von 40 bis 50 mg/ g Zementpulver.

7. Kit-of-Parts, enthaltend im wesentlichen die folgenden getrennt gepackten Komponenten: (i) eine definierte Menge von Knochenzementpulver auf Basis von α-BSM, vermengt mit Gentamycinsulfat, Tobramycinsulfat, Amikacinsulfat oder Netilmycinsulfat in einer biologisch wirksamen Konzentration zwischen 40 und 50 mg/g Zementpulver ggf. in Anwesenheit eines Stabilisierungs-, Konservierungs-, Binde- und/oder Komplexierungsmittels und (ii) eine darauf abgestimmte Flüssigkeitsmenge, die so gewählt ist, dass nach Zusammenbringen der beiden Komponenten (i) und (ii) eine leicht injizierbare aushärtbare Paste entsteht.

## Claims

1. Injectable, curable bone-cement paste based on bioabsorbable α-BSM comprising a medicament having a certain concentration, **characterised in that** the medicament is gentamycin sulfate, tobramycin sulfate, amikacin sulfate or netilmycin sulfate in a concentration of from 40 to 50 mg/g of cement powder.

2. Active-ingredient depot comprising a bone-cement paste defined in accordance with Claim 1 for the continuous release of the antibiotic gentamycin sulfate, tobramycin sulfate, amikacin sulfate or netilmycin sulfate in a concentration of from 40 to 50 mg/g of cement powder and over an extended period for the treatment and prophylaxis of osteomyelitis and osteitis, in particular as a consequence of bone defects or fractures.

3. Use of a bone-cement powder based on bioabsorbable α-BSM together with gentamycin sulfate, tobramycin sulfate, amikacin sulfate or netilmycin sulfate for the preparation of an injectable, curable, bioabsorbable, hydroxylapatite-like bone-cement paste having the ability to release the antibiotic after curing in vivo in a biologically effective concentration of from 40 to 50 mg/g of cement powder and over an extended period, for the treatment and prophylaxis of osteomyelitis and osteitis, in particular as a consequence of bone defects or fractures.

4. Process for the preparation of an injectable, curable bone-cement paste based on bioabsorbable α-BSM, comprising gentamycin sulfate, tobramycin sulfate, amikacin sulfate or netilmycin sulfate, **characterised in that** bone-cement powder based on α-BSM is mixed with an aqueous solution of the antibiotic, if desired with addition of stabilisers, preservatives, binders and/or complexing agents.

5. Kit of parts essentially containing the following separately packed components: (i) a defined amount of bone-cement powder based on α-BSM, and (ii) a matched amount of liquid comprising gentamycin sulfate, tobramycin sulfate, amikacin sulfate or netilmycin sulfate in a biologically effective concentration of from 40 to 50 mg/g of cement powder, if desired in the presence of stabilisers, preservatives, binders and/or complexing agents, where the amount of liquid is selected in such a way that, after the two components (i) and (ii) have been combined, a readily injectable, curable paste is formed.

6. Kit of parts according to Claim 5, containing the said components in the following units: 1 g of (i) and 0.7 ml of (ii) having a variable antibiotic content of from 40 to 50 mg/g of cement powder.

7. Kit of parts essentially containing the following separately packed components: (i) a defined amount of bone-cement powder based on α-BSM mixed with gentamycin sulfate, tobramycin sulfate, amikacin sulfate or netilmycin sulfate in a biologically effective concentration of between 40 and 50 mg/g of cement powder, if desired in the presence of a stabiliser, preservative, binder and/or complexing agent, and (ii) a matched amount of liquid which is selected in such a way that, after the two components (i) and (ii) have been combined, a readily injectable, curable paste is formed.

## Revendications

1. Pâte de ciment d'os injectable et durcissable, à base d'α-BSM [Matériau de Substitution d'Os] biorésorbable, comprenant un médicament ayant une certaine concentration, **caractérisée en ce que** le médicament est le sulfate de gentamycine, le sulfate de tobramycine, le sulfate d'amikacine ou le sulfate de nétilmycine en une concentration allant de 40 à 50 mg/g de poudre de ciment.

2. Ingrédient actif retard comprenant une pâte de ciment d'os définie conformément à la revendication 1, pour la libération continue de l'antibiotique : le sulfate de gentamycine, le sulfate de tobramycine, le sulfate d'amikacine ou le sulfate de nétilmycine, en une concentration allant de 40 à 50 mg/g de poudre de ciment, et au cours d'une période étendue pour le traitement et la prophylaxie de l'ostéomyélite et de l'ostéite, en particulier comme conséquence de défauts ou de fractures d'os.

3. Utilisation d'une poudre de ciment d'os à base d'α-BSM biorésorbable, conjointement avec le sulfate de gentamycine, le sulfate de tobramycine, le sulfate d'amikacine ou le sulfate de nétilmycine pour la préparation d'une pâte de ciment d'os injectable, durcissable, biorésorbable, semblable à de l'hydroxyapatite et étant capable de libérer l'antibiotique après un durcissement in vivo en une concentration biologiquement efficace allant de 40 à 50 mg/g de poudre de ciment, et au cours d'une période étendue, pour le traitement et la prophylaxie de l'ostéomyélite et de l'ostéite, en particulier comme conséquence de défauts ou de fractures d'os.

4. Procédé de préparation d'une pâte de ciment d'os injectable, durcissable et à base d'α-BSM biorésorbable, comprenant du sulfate de gentamycine, du sulfate de tobramycine, du sulfate d'amikacine ou du sulfate de nétilmycine, **caractérisé en ce que** la poudre de ciment d'os à base d'α-BSM est mélangée avec une solution aqueuse de l'antibiotique, si on le désire avec l'addition d'agents stabilisants, conservateurs, liants et/ou complexants.

5. Kit de pièces contenant essentiellement les composants suivants emballés séparément : (i) une quantité définie de poudre de ciment d'os à base d'α-BSM, et (ii) une quantité identique de liquide comprenant du sulfate de gentamycine, du sulfate de tobramycine, du sulfate d'amikacine ou du sulfate de nétilmycine en une concentration biologiquement efficace allant de 40 à 50 mg/g de poudre de ciment, si on le désire en présence d'agents stabilisants, conservateurs, liants et/ou complexants, dans lequel la quantité de liquide est choisie de telle sorte que, après l'association des deux composants (i) et (ii), une pâte facilement injectable et durcissable soit formée.

6. Kit de pièces selon la revendication 5, contenant lesdits composants selon les unités suivantes: 1 g de (i) et 0,7 ml de (ii) ayant une teneur variable en antibiotique allant de 40 à 50 mg/g de poudre de ciment.

7. Kit de pièces contenant essentiellement les composants suivants emballés séparément : (i) une quantité définie de poudre de ciment d'os à base d'α-BSM mélangée avec du sulfate de gentamycine, du sulfate de tobramycine, du sulfate d'amikacine ou du sulfate de nétilmycine en une concentration biologiquement efficace comprise entre 40 et 50 mg/g de poudre de ciment, si on le désire en présence d'un agent stabilisant, conservateur, liant et/ou complexant, et (ii) une quantité identique de liquide qui est choisie de telle sorte que, après l'association des deux composants (i) et (ii), une pâte facilement injectable et durcissable soit formée.
